(19) Europäisches Patentamt
European Patent Office
Office européen des brevets

(11) Publication number: 0 214 009
A2

(12) # EUROPEAN PATENT APPLICATION

(21) Application number: 86401678.7

(22) Date of filing: 28.07.86

(51) Int. Cl.[4]: C 07 C 101/28
C 07 D 307/66,
C 07 D 309/32,
A 61 K 31/215,
A 61 K 31/365

(30) Priority: 30.07.85 US 760423

(43) Date of publication of application: 11.03.87 Bulletin 87/11

(84) Designated Contracting States: CH DE FR GB IT LI NL

(71) Applicant: MERCK & CO. INC.
126, East Lincoln Avenue P.O. Box 2000
Rahway New Jersey 07065 (US)

(72) Inventor: Naringrekar, Vijay H.
12F Edison Court
Monsey New York 10952 (US)

Stella, Valentino J.
77 Sunset Drive
Lawrence Kansas 66044 (US)

(74) Representative: Ahner, Francis et al
CABINET REGIMBEAU 26, avenue Kléber
F-75116 Paris (FR)

(54) **Enaminones as potential prodrugs of primary and secondary amines.**

(57) Novel enzymatically labile enaminones useful as potential prodrugs of primary and secondary amines are disclosed. Also, disclosed herein are compositions comprising said amines and methods of administering said composition to warm-blooded animals.

EP 0 214 009 A2

## Description

# ENAMINONES AS POTENTIAL PRODRUGS OF PRIMARY AND SECONDARY AMINES

## BACKGROUND OF THE INVENTION

The invention relates to novel and useful derivatives of enaminone prodrugs containing a primary or secondary amine function. More particular, the invention relates to novel forms of these drugs characterized as being (I) more readily bioavailable; (2) less irritating to topical and gastric mucosal membranes; and (3) more permeable through topical membranes such as the ophthalmic membrane, skin, or the buccal, rectal or gut mucosa and the like, when administered orally, intravenously or topically to warm-blooded animals than are the drugs from which they are derived.

This invention describes enzymatically labile enaminones as potentially useful prodrugs of drugs containing primary or secondary amino groups. The presence of a primary or secondary amino group in a drug molecule may deleteriously affect its physico-chemical properties such as stability and solubility. As peptidases, responsible for either terminal amino acid cleavage or cleavage of a lysine residue, use the primary or secondary amino group as a recognition factor, it may be possible to slow or suppress this breakdown, in part, by making suitable prodrugs that mask said amino groups.

For the purposes of this specification, the term "prodrug" denotes a derivative or primary or secondary amine compound in particular known primary and secondary amine containing drugs (methyldopa, timolol, penicillamine, and the like) which derivative, when administered to warm-blooded animals, "cleaves" in such a manner as to release the drug form at its target site or sites of activity. The enzymatic and/or chemical hydrolytic "cleavage" of the compounds of the invention occurs in such a manner such that the proven drug form (the primary or secondary amine compound) is released while the remaining "cleaved" moiety remains nontoxic and is metabolized in such a manner that nontoxic, metabolic products are produced.

## DESCRIPTION OF THE PRIOR ART

Usually, the un-ionized form of a drug is absorbed more efficiently than its ionic species. In the case of drugs containing a primary or secondary amine group such as methyldopa, penicillamine, and the like, the amine function is significantly ionized at physiological pH. The result is that such drugs are poorly absorbed through lipid-water membrane barriers and are irritating. Thus, an object of the present invention is to provide a class of derivatives of drugs which would not be significantly ionized at physiological pH and which are reasonably stable but which hydrolyze readily in vivo.

The prodrugs of this invention have generally lower melting points and better lipid water diphasic solubility which enable them to penetrate biological membranes such as the skin or the buccal, rectal or gut mucosa better than the parent drug. The prodrug is then cleaved chemically or enzymatically to deliver the parent drug in vivo.

## SUMMARY OF THE INVENTION

The invention relates to novel and useful derivatives of enaminone prodrugs containing a primary or secondary amine function.

Accordingly, it is an object of the invention to provide enaminone prodrug forms of primary and secondary amine drugs which exhibit pharmaceutical activity when administered to warm-blooded animals and are characterized as being more readily bioavailable and less irritating to topical and gastric mucosal membranes such as the skin, or the buccal, rectal or gut mucosa and more permeable through topical membranes, e.g., ophthalmic membrane and skin, than are the parent drugs from which they are derived.

It is another object of the present invention to provide such prodrug form of primary and secondary amine compounds which, following administration, will "cleave" in such a manner as to enable the original parent moiety to be released at its therapeutic site or sites of activity and to further permit the cleaved moiety unassociated with the parent moiety to be metabolized in a nontoxic fashion.

Another object of the invention is to provide prodrugs of medicaments or drugs having primary or secondary amine functions thereon to provide increased biomembrane transport such that the drug is more bioavailable from the GI tract, the rectum, the skin and the eye of the human body.

These and other objects and advantages of the invention are achieved by administering to warm-blooded animals afflicted with a particular disease or disorder against which the parent drug is effective when administered in a therapeutically effective amount will become apparent from the detailed description.

## DETAILED DESCRIPTION OF THE INVENTION

The invention concerns the preparation and use of novel enaminone prodrugs having primary or secondary amine functions which increases the bioavailability of said prodrug when administered to warm-blooded animals. The novel enaminone prodrugs of the invention are represented by the following formulae:

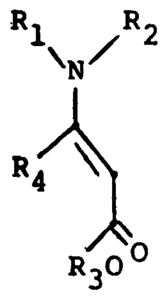

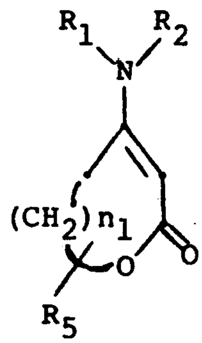

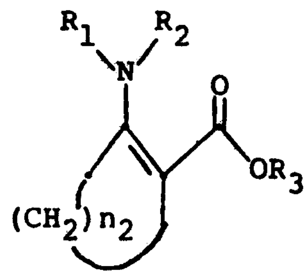

I II III

wherein $n_1$ is I or 2; $n_2$ is 3 or 4; $R_1R_2N$-H is any drug, pharmaceutical or medicament having a primary or secondary amine function; $R_3$ and $R_4$ can be the same or different and is alkyl ($C_1$-$C_6$) such as methyl, ethyl, t-butyl and the like, aryl ($C_6$-$C_{10}$) such as phenyl; aralkyl ($C_7$-$C_{12}$) such as benzyl, tolyl and the like; cycloalkyl ($C_3$-$C_8$) such as cyclohexyl, cyclopentyl, cyclooctyl and the like; cycloalkenyl ($C_3$-$C_{10}$) such as cyclohexenyl, cyclopropenyl, cyclodecenyl and the like; alkenyl ($C_2$-$C_{20}$) such as allyl, butenyl, octenyl, decenyl, heradecenyl and the like; saturated or unsaturated mono- or polyheterocyclics having from I to 3 rings and having one or more nitrogen, oxygen or sulfur atoms in the rings such as furyl, morpholinyl, oxazolidinyl, pyrolyl, I,2,5-thiazolyl, I-pyrrolidinyl, 4-methyl-I-piperazinyl, piperidino, imidazolyl, I-pyrazolyl and the like; and $R_5$ is hydrogen or alkyl ($C_1$-$C_8$) such as methyl, ethyl isopropyl hexyl and the like.

The process for preparing compounds of formulae I, II or III is known in the art and generally involves a one-step reaction. However, in some examples (see No. 2), multi-conventional reaction steps are employed. See the general reaction scheme for Ethyl 2-(N-phenylamino)-I-cyclohexene-I-carboxylate below:

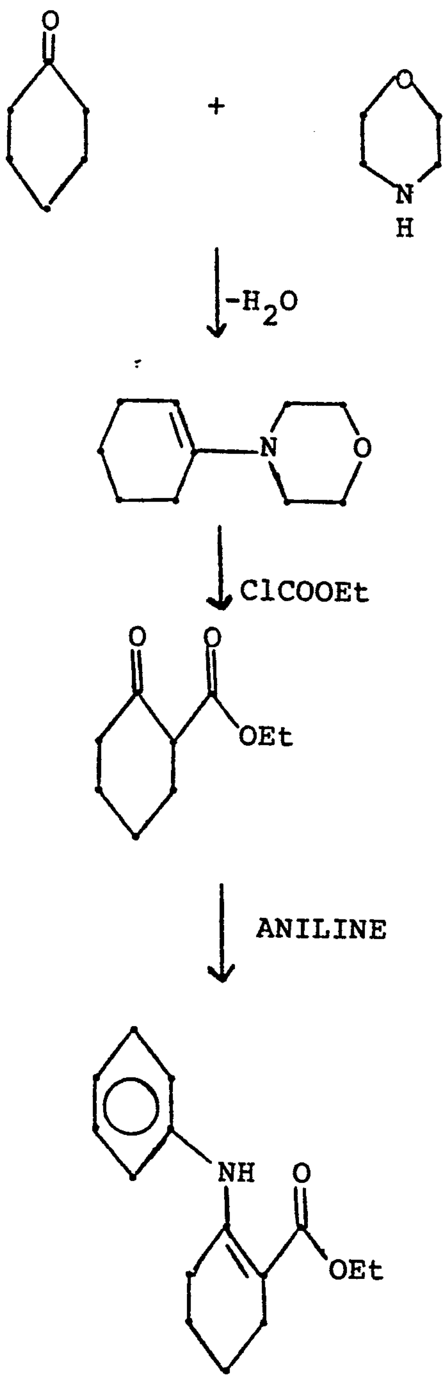


Enaminones of the type shown in Figure I are a class of enamine and may be considered as products of a reaction between a primary amine and a I,3-dicarbonyl compound. Like most other enamines, enaminones are chemically unstable under acidic pH conditions and relatively stable under neutral to mildly alkaline pH conditions. This makes them potentially unsuitable as prodrugs for the oral delivery of amine drugs. We have performed a systematic study concerning the structure - stablility relationship for enaminones. It was observed that the stability of enaminones was relatively independent of the $pk_a$ of the amine used to form the enaminone, but was extremely sensitive to the structure of the dicarbonyl compounds used to form the enaminones. Cyclic dicarbonyl compounds in particular produced relatively more stable enaminones compared to acyclic dicarbonyl compounds. We have found that enaminones are relatively stable under acidic pH conditions, although they are usually extremely stable under physiological pH conditions.

Enaminones are relatively more stable than simple enamines due to the extended conjugation of the vinylic double bond with the carbonyl group. This generalization holds whether the carbonyl group is from a ketone or an ester function. As indicated in Scheme I above, if the ester bond of I, II or III is cleaved by esterases, the keto carboxylate-type enaminone formed, should chemically hydrolyze faster than I, II or III because of the significant reduction in the $\pi$-bond overlap responsible for the stabilization of the enaminones is lost after

ester hydrolysis. This hypothesis was tested for two enaminones, ethyl 3-(N-phenylamino)-2-butenoate, (Example 3), and ethyl 2-(N-phenylamino)-l-cyclopentene-l-carboxylate (Example 2).

Enaminones (Examples 2 and 3) were synthesized according to the method of Reynolds and Hauser, Organic Synthesis Collective III, 374-75 and their hydrolysis at 25°C, in isotonic Sorensen phosphate buffer, pH 7.4, was studied spectrophotometrically at 298.5 nm for Example 3 and 315.5 nm for Example 2 (their respective max) in the presence and absence of various enzyme systems. Porcine liver esterase (Sigma *E-3128, Type I) appeared to catalyze the hydrolysis of Examples 3 and 2 relative to its chemical hydrolysis eg. at pH 7.4 and 25°C, the apparent half lives for chemical hydrolysis of Examples 2 and 3 were 18 min. and 15.5 hours, respectively. In the presence of 60 × $10^{-4}$ units/ml activity of porcine liver esterase and an initial substrate concentration of 16 μM, the apparent half lives were reduced to 2 minutes and 10 minutes for Examples 3 and 2, respectively. Apparent Michaelis-Menten kinetics was observed. The Michaelis-Menten parameters for the hydrolysis of said Examples 3 and 2 from initial hydrolysis rates fitted by Lineweaver Burk plots, are disclosed in Tables I and II below, respectively.

## TABLE I

$V_{max}$ and $K_m$ values for the hydrolysis of ethyl-3-(phenylamino)-2-butenoate in the presence of porcine liver esterase.

| Enzyme Concentration Units/ml x $10^4$ | Vmax ± S.D. $mM.h^{-1}$ | Km ± S.D. mM |
|---|---|---|
| 16.7 | 84 ± 3 | 6.0 ± 0.1 |
| 32.4 | 233 ± 20 | 8.5 ± 0.2 |
| 61.2 | 485 ± 83 | 13.0 ± 0.3 |

Average $K_m$ = 9.2 ± 2.9 mM

## TABLE II

$V_{max}$ and $K_m$ values for the hydrolysis of ethyl-2-(N-phenylamino)-1-cyclohexene-1-carboxylate in the presence of porcine liver esterase.

| Enzyme Concentration Units/ml x $10^4$ | Vmax ± S.D. $mM.h^{-1}$ | Km ± S.D. mM |
|---|---|---|
| 16.7 | 45.5 ± 3 | 11.2 ± 0.1 |
| 32.4 | 56.8 ± 8 | 5.3 ± 0.2 |
| 61.2 | 95.2 ± 10 | 5.2 ± 0.2 |

Average $K_m$ = 7.2 ± 2.8 mM

No catalysis by porcine liver esterase was observed for hydrolysis of 4-(N-phenylamino)-3-pentene-2-one (Example 4), an enaminone structurally similar to Example 3 but without the ester function eg. the half-life for hydrolysis of Example 4 was the same ( 34 hours) in presence and absence of porcine liver esterase. Hence it appears that the presence of the ester group was necessary for the catalytic effect by porcine liver esterase. Aniline, as expected, was observed to be a product of both the chemical and enzyme catalyzed hydrolysis. The corresponding keto-esters were the other products of the chemical hydrolysis. Although the second product of the enzyme catalyzed hydrolysis could not be directly identified, it was not the corresponding keto-ester. Thus, it appears, as expected, that the ketoester is hydrolysed, prior to enaminone hydrolysis. Catalysis of the hydrolysis of ethyl-3-(N-phenylamino)-2-butenoate and ethyl-2-(N-phenylamino)-l-cyclohexene was not observed when studies were carried out in the presence of rat and human plasma, rat liver and intestinal homogenates and carbonic anhydrase. Thus, it appears that the keto-ester-type enaminones are enzymatically labile but their enzymatic lability is limited to some specific enzyme(s) that do not appear to be ubiquitous.

This appears to be the first time that enzyme-aided hydrolysis of enaminones has been observed, suggesting that it may be possible to utilize chemically stable keto-ester-type enaminones as prodrugs of primary amino group containing drugs. If keto-ester-type enaminones with desired chemical stability characteristics are synthesized, they may be subject to enzyme-aided hydrolysis via the mechanism that releases the parent amine drug in the body.

$R_1R_2NH$ can represent any drug, pharmaceutical or medicament having a primary or secondary amine function thereon. Typical drugs, pharmaceuticals or medicaments that can be used and have a primary or secondary amine function thereon are timolol, norfloxacin, dimethoxyphenethylamine, propranolol, atenolol, pindolol, betaxalol, methyldopa and the like.

Various active agents provide beneficial effects when administered to patients. Representative drugs, pharmaceuticals or medicaments which can be used and which contain primary or secondary amine functions thereon are listed below. One skilled in the art will realize that the list below is not exclusive and the invention is applicable to other primary and secondary amino functional drugs as well.

a) Those drugs, pharmaceuticals or medicaments similar to timolol: acebutalol, albuterol, alprenolol, atenolol, bucindolol, bunolol, butopamine, butoxamine, carbuterol, cartelolol, colterol, deterenol, dexpropranolol, diacetolol, dobutamine, exaprolol, fenoterol, fenyripol, labotolol, levobunolol, metolol,

metaproterenol, metoprolol, nadolol, exprenolol, pamatolol, penbutalol, pindolol, pirbuterol, practolol, prenalterol, primidolol, prizidilol, procaterol, propranolol, quinterenol, rimiterol, ritodrine, sotolol, soterenol, sulfinolol, sulfonterol, suloctidil, tazolol, terbutaline, tiprenolol, tipropidil, tolamolol, etc.

b) Structurally similar to thiabendazole: albendazole, albutoin, alinidine, alizapride, amiloride, aminorex, aprinocid, cambendazole, cimetidine, clonidine, cyclobendazole, etintidine, fenbendazole, fenmetazole, flubendazole, fludorex, lobendazole, mebendazole, metazoline, nocodazole, oxfendazole, oxibendazole, oxmetidine, parbendazole, ranitidine, tetrahydrazoline, tiamenidine, tinazoline, tiotidine, tolazoline, tramazoline, xylometazoline, etc.

c) Structurally similar to dimethoxyphenethylamine: adrenelone, aletamine, amidephrine, amphetamine, aspartame, bamethan, betahistine, clorprenaline, chlortermine, dopamine, etryptamine, fenfluramine, norepinephrine, tocainide, etc.

Other drugs are acyclovir, enviroxime, etoprine, nifedipine, nimodipine, triamterene, vidarabine, methyldopa, epinephrine and those structurally similar to norfloxacin such as pipemidic acid, l-ethyl-6-fluoro-l,4-dihydro-4-oxo-7-(l-piperazinyl)-l,8-naphthyridine-3-carboxylic acid and l-cyclopropyl-6-fluoro-l,4-dihydro-4-oxo-7-(l-piperezinyl)-3-quinolinecarboxylic acid.

The prodrug compounds of the invention can be used to treat any condition for which the parent drug, medicament or pharmaceutical is useful. For example, if timolol is the parent drug of choice, the prodrug can be used for any condition or treatment for which timolol would be administered.

Thus, the prodrug compounds of Formulae (I) and II may be administered orally, topically, parentally, by inhalation spray or rectally in dosage unit formulations containing conventional, non-toxic pharmaceutically acceptable carriers, adjuvants and vehicles.

The pharmaceutical compositions containing the active ingredient may be in a form suitable for oral use, for example, as tablets, troches, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparation.

Formulations for oral use include tablets which contain the active ingredient in admixture with non-toxic pharmaceutically acceptable excipients. These excipients may be, for example, inert diluents, such as calcium carbonate, sodium chloride, lactose, calcium phosphate or sodium phosphate; granulating and disintegrating agents, for example, maize starch, or alginic acid; binding agents, for example, starch, gelatin or acacia, and lubricating agents, for example, magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example, peanut oil, liquid paraffin, or olive oil.

Aqueous suspensions usually contain the active materials in admixture with appropriate excipients. Such excipients are suspending agents, for example, sodium carboxymethylcellulose, methyl cellulose, hydroxypropylmethylcellulose, sodium alginate, polyvinylpyrrolidone, gum tragacanth and gum acacia; dispersing or wetting agents which may be a naturally-occurring phosphatide, for example, lecithin; a condensation product of an alkylene oxide with a fatty acid, for example, polyoxyethylene stearate; a condensation product of ethylene oxide with a long chain aliphatic alcohol, for example, heptadecaethyleneoxycetanol; a condensation product of ethylene oxide with a partial ester derived from fatty acids and a hexitol.such as polyoxyethylene sorbitol monooleate; or a condensation product of ethylene oxide with a partial ester derived from fatty acids and hexitol anhydrides, for example, polyoxyethylene sorbitan monooleate. The aqueous suspensions may also contain one or more preservatives, for example, ethyl, n-propyl, or p-hydroxybenzoate; one or more coloring agents; one or more flavoring agents; and one or more sweetening agents such as sucrose or saccharin.

Oily suspension may be formulated by suspending the active ingredient in a vegetable oil, for example, arachis oil, olive oil, sesame oil or coconut oil, or in a mineral oil such as liquid paraffin. The oily suspensions may contain a thickening agent, for example, beeswax, hard paraffin or cetyl alcohol. Sweetening agents such as those set forth above, and flavoring agents may be added to provide a palatable oral preparation. These compositions may be preserved by the addition of an antioxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example, sweetening, flavoring and coloring agents, may also be present.

The pharmaceutical compositions of the invention may also be in the form of oil-in-water emulsions. The oily phase, may be a vegetable oil, for example, olive oil or arachis oils, or a mineral oil, for example, liquid paraffin or mixtures of these. Suitable emulsifying agents may be naturally-occurring gums, for example, gum acacia or

gum tragacanth; naturally-occurring phosphatides, for example, soybean lecithin; and esters including partial esters derived from fatty acids and hexitol anhydrides, for example, sorbitan mono-oleate, and condensation products of the said partial esters with ethylene oxide, for example, polyoxyethylene sorbitan monooleate. The emulsions may also contain sweetening and flavoring agents.

Syrups and elixirs may be formulated with sweetening agents, for example glycerol, sorbitol or sucrose. Such formulations may also contain a demulcent, a preservative and flavoring and coloring agents. The pharmaceutical compositions may be in the form of a sterile injectable aqueous or oleagenous suspension. This suspension may be formulated according to the known art using those suitable dispersing or wetting agents and suspending agents which have been mentioned above. The sterile injectable preparation may be a sterile injectable solution or suspension in a non-toxic parenterally-acceptable diluent or solvent. Among the acceptable vehicles and solvents that may be employed are water, l,3-butanediol, Ringer's solution and isotonic sodium chloride solution. In addition, sterile fixed oils are conventionally employed as a solvent or suspending medium. For this purpose any bland fixed oil may be employed including synthetic mono- or diglycerides. Fatty acids such as oleic acid also find use in the preparation of injectibles.

The prodrug compounds of the invention may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug, for example, cocoa butter and polyethylene glycols.

For topical use, creams, ointments, jellies, solutions, suspensions or the like containing the prodrugs are employed according to methods recognized in the art.

Naturally, the therapeutic dosage range for the compounds of the invention will vary with the size and needs of the patient and the particular pain or disease symptom being treated. However, generally speaking, the following dosage guidelines will suffice. Orally, the therapeutic dose required for a compound of the invention will generally, on a molecular basis, mimic that for the parent primary or secondary amine drug. On a topical basis, application of a 0.0l% to 2.5% concentration of a compound of the invention (in a suitable topical carrier material) to the affected site should suffice.

The amount of active ingredient that may be combined with the carrier materials to produce a single dosage form will vary depending upon the host treated and the particular mode of administration. For example, a formulation intended for the oral administration of humans may contain from 5 mg to 5 gm of active agent compounded with an appropriate and convenient amount of carrier material which may vary from about 5 to about 95 percent of the total composition. Other dosage forms such as ophthalmic dosage forms contain less active ingredient such as for example from 0.l mg to 5 mg. Dosage unit forms will generally contain between from about 0.l mg to about l g of active ingredient.

It will be understood, however, that the specific dose level for any particular patient will depend upon a variety of factors including the activity of the specific compound employed, the age, body weight, general heath, sex, diet, time of administration, route of administration, rate of excretion, drug combination and the severity of the particular disease undergoing therapy.

The following examples illustrate preparation of various enaminone prodrugs of the invention. The examples should be construed as illustrations rather than limitations thereof.

EXAMPLE I

Ethyl 2-oxo-cyclohexane-l-carboxylate

This compound was synthesized from morpholine and cyclohexanone, using the literature procedure of Stork et al., J. Am. Chem. Soc., 85, 207-222 (l963). The intermediate, l-(N-morpholino)-l-cyclohexene was purified by vacuum distillation and the structure confirmed by pmr spectra. Yield: 87.2%. Also, the intermediate l-(N-morpholino)-l-cyclohexene was reacted with ethyl chloroformate to obtain ethyl 2-oxo-cyclohexane-l-carboxylate in overall yield of 40%. The structure was confirmed by pmr and used as reagent in Example 2.

EXAMPLE 2

Ethyl 2-(N-phenylamino)-l-cyclohexene-l-carboxylate

This compound was synthesized by the method reported by Taguchi and Westheimer, J. Organic Chemistry, 36, l570-l572 (l97l). A solution of 8.5 g (0.05 mole) obtained from Example l and 5.5 ml (0.06 mole) freshly distilled aniline, in 20 ml dry benzene was stirred at room temperature for l4 hours, in the presence of 20 g molecular sieves (Linde 4A). The mixture was filtered. The filtrate was subjected to rotary evaporation to give a thick reddish residue. The residue was purified by treatment with decolorizing charcoal and crystallization from petroleum ether (20-40° C) to give white crystals.

Yield: 5 g (40.8%); m.p., 57-58° C.
Anal.---calc. for $C_{15}H_{19}NO_2$:
C, 73.47; H, 7.76; N, 5.7l.
Found: C, 73.28; H, 8.l0; N, 5.82.
PMR:(CDCl3) l.29 (t, 3H, $CH_3$-C, J=7 Hz), l.59 (m, 4H, C-$CH_2CH_2$-C), 2.35 (broad s, 4H, $CH_2$-C=C-), 4.l7 (g, 2H, $CH_2O$), 7.l4 (m, 5H, Ar-H), l0.73 (broad s, lH, NH) ppm.

EXAMPLE 3

Ethyl 3-(N-phenylamino)-2-butenoate

This compound was prepared according to the method of Reynolds and Hauser, Organic Synthesis Collective Volume III, 374-75 (1955). A solution of 6.4 ml (0.05 mole) ethyl aceto-acetate, 4.6 ml (0.05 mole) freshly distilled aniline and 0.1 ml glacial acetic acid in 10 ml benzene was refluxed in a 50 ml round-bottomed flask attached to a Dean and Stark constant water separator. Refluxing was continued till no more water separated (3 hours). Benzene was then removed by rotary evaporation and the product was purified by vacuum distillation. Yield, 7.5 g (73%).

Anal.---calc. for $C_{12}H_{15}NO_2$:

C, 70.24; H, 7.32; N, 6.83.

Found: C, 69.60; H, 7.60; N, 6.70.

PMR: ($CDCl_3$) 1.26 (t, 3H, $CH_3C$, J=7 Hz), 1.96 (s, 3H, $CH_3C=C$), 4.14 (g, 2H, $CH_2$-C, J=7 Hz), 4.69 (s, 1H, CH=C), 7.21 (m, 5H, Ar-H), 10.38 (broad s, 1H, NH) ppm.

EXAMPLE 4

3-[1-(N-Phenylamino)-ethylidene]-4,5-dihydro-2(3H)-furanone

This compound was synthesized by the general method for enamine synthesis of Stork et al., J. Am. Chem. Soc., 76, 2029-2030 (1954). The method was essentially similar to that described for Example 3, except that aniline was reacted with 3-acetyl-4,5-dihydro-2(3H)-furanone and the glacial acetic acid was excluded. The product was recrystallized from cyclohexane. Yield, 80%; m.p., 87-88°C.

Anal.---calc. for $C_{12}H_{13}NO_2$:

C, 70.93; H, 6.4; N, 6.89.

Found: C, 70.53; H, 6.68; N, 6.59.

PMR: ($CDCl_3$) 2.01 (s, 3H, $CH_3C=C$), 2.89 (t, 2H, $CH_2C=C$, J=7.8 Hz), 4.34 (t, 2H, $CH_2O$, J=7.8 Hz), 7.17 (m, 5H, Ar-H), 9.97 (broad s, 1H, NH) ppm.

EXAMPLE 5

4-(N-Phenylamino)-3-pentene-2-one

This compound was prepared by the method of Stork et al., J. Am. Chem. Soc., 76, 2029-2030 (1954) as described in Example 3, by the reaction of aniline with pentane-2,4-dione. The product was purified by recrystallization from petroleum ether (40-60°C).

Yield, 94%; m.p., 42-43°C.

Anal.---calc. for $C_{11}H_{13}$ NO:

C, 75.43; H, 7.43; N, 8.00.

Found: C, 75.70; H, 7.70; N, 7.90.

PMR: ($CDCl_3$) 1.99 (s, 3H, $CH_3C=C$), 2.09 (s, 3H, $CH_3C=O$), 5.20 (s, 1H, CH=C), 7.39 (m, 5H, Ar-H), 12.46 (broad s, 1H, NH) ppm.

EXAMPLE 6

Ethyl 2-(N-Phenylamino)-1-cyclopentene-1-carboxylate

A procedure similar to that described in Example 3 was used by reacting aniline with ethyl 2-oxo-cyclopentane-1-carboxylate. The product was purified by column chromatography (alumina, 20% acetone in n-hexane).

Anal.---calc. for $C_{14}H_{17}NO_2$:

C, 72.72; H, 7.36; N, 6.06.

Found: C, 73.15; H, 8.20; N, 6.00.

PMR: ($CDCl_3$) 1.29 (t, 3H, $CH_3C$, J=7 Hz), 1.93 (m, 2H, C-$CH_2$-C, J=7 Hz), 2.69 (m, 4H, $CH_2$-C=C, J=7 Hz), 4.2 (g, 2H, $OCH_2$, J=7 Hz), 7.06 (m, 5H, Ar-H), 9.59 (broad s, 1H, NH) ppm.

EXAMPLE 7

4-(2-N-Phenethylamino)-3-pentene-2-one

β-Phenylethylamine was reacted with pentane-2,4-dione by the method described in Example 4. The product was purified by vacuum distillation. Yield, 80%.

Anal.---calc. for $C_{13}H_{17}NO$:

C, 76.85; H, 8.37; N, 6.9.

Found: C, 75.10; H, 8.50; N, 6.62.

PMR:($CDCl_3$) 1.74 (s, 3H, $CH_3C=C$), 1.96 (s, 3H, $CH_3C=O$), 2.8 (t, 2H, $CH_2$-Ar, J=7.0 Hz), 3.40 (m, 2H, $CH_2$-N, J=6.8 Hz), 7.22 (broad s, 5H, Ar-H), 10.88 (broad s, 1H, NH) ppm.

EXAMPLE 8

3-(N-Phenylamino)-2-cyclohexene-l-one

This compound was synthesized by the same method as that described in Example 4, by reacting aniline with cyclohexane-2.4-dione. The product was recrystallized from methanol. Yield, 94%; m.p., l78-l82° C.

Anal.---calc. for $C_{12}H_{13}NO$:

C, 77.00: H, 6.95: N, 7.49.

Found: C. 76.79: H. 7.20: N, 6.99.

PMR: ($CDCl_3$) 2.02 (m, 2H, C-$CH_2$-C, J=5.8 Hz), 2.3l (t, 2H, $CH_2C$=C, J=6.0 Hz), 2.5 (t, 2H, $CH_2C$=O, J=5.9 Hz), 5.53 (s, lH, CH=C), 7.l7 (m, 5H, Ar-H), 7.43 (broad s, lH, NH) ppm.

EXAMPLE 9

5,6-Dihydro-6-(l-methylethyl)-4-(N-phenylamino)-2-pyrone

5,6-Dihydro-4-hydroxy-6-(l-methylethyl)-2-pyrone (8a) and Methyl 5-hydroxy-6-methyl-3-oxoheptane-l-carboxylate (l5a) were prepared from isobutyraldehyde and diketene, by the method reported by Izawa and Mukaiyama, Chem. Lett., l6l-l64 (l975). A mixture of 8a and l5a was separated and isolated by the work-up procedure described by Izawa and Mukaiyama. 8a was obtained in 25.6% yield (m.p., 78.5-79.5° C) and l5a was isolated as a viscous liquid in 64% yield. The structures of 8a and l5a were confirmed by pmr.

5,6-Dihydro-6-(l-methylethyl)-4-(N-phenylamino)-2-pyrone was prepared by reacting 8a with aniline in 70% yield by the standard method described earlier for compound l; m.p. l95-l96° C.

Anal.---calc. for $C_{14}H_{17}NO_2$:

C, 72.72: H, 7.36: N, 6.06.

Found: C, 72.l0; H, 7.60; N, 6.20.

PMR: ($CDCl_3$) l.03 (d, 6H, $(CH_3)_2CH$, J=3 Hz),

l.8l (m, lH, $CHC_3$), 2.52 (m, 2H, $CH_2$-C=C), 3.69 (m, lH, CH-O), 5.23 (s, lH, C=CH-C=O), 6.82 (s, lH, NH), 7.25 (m, 5H, Ar-H) ppm.

EXAMPLE l0

5,6-Dihydro-6-methyl-4-(N-phenylamino)-2-pyrone

5,6-Dihydro-4-hydroxy-6-methyl-2-pyrone (9a) was prepared by method identical to that used ro 8a in Example 9 with paraldehyde substituted for isobutyraldehyde. Yield, 75%; m.p., l22-l23° C. The structure was confirmed by pmr. The resulting product was reacted with aniline by the method similar to that for l to obtain 9 in 63% yield, m.p., l90-l9l C.

Anal.---calc. for $C_{12}H_{13}NO_2$: C, 70.93; H, 6.40; N, 6.89.

Found: C, 70.00: H, 6.33: N, 6.95.

PMR: ($CDCl_3$) l.35 (d, 3H, $CH_3C$, J=7 Hz), 2.46 (m, 2H, $CH_2$-C=C), 4.40 (m, lH, CH-O), 5.l7 (s, lH, CH=C), 7.l5 (m, 5H, Ar-H), 7.27 (s, lH, NH) ppm.

EXAMPLE ll

5,5-Dimethyl-3-(N-phenylamino)-2-cyclohexene-l-one

The standard method described in Example 4 was followed to synthesize the title compound from aniline and 5,5-dimethylcyclohexane-l,3-dione. The final product crystallized out of the reaction mixture in 9l% yield, m.p. l86-l88° C.

Anal.---calc. for $C_{14}H_{17}NO$: C, 78.l4; H, 7.9l; N, 6.5l.

Found: C, 78.65: H, 8.25: N, 6.l0.

PMR: ($CDCl_3$) l.04 (s, 6H, $(CH_3)_2C$), 2.l6 (s, 2H, $CH_2C$=C), 2.34 (s, 2H, $CH_2C$=O), 5.53 (s, lH, CH=C), 7.l5 (m, 5H, Ar-H), 7.30 (broad s, lH, NH) ppm.

EXAMPLE l2

4-(N-Phenylamino)-2(5H)-furanone

This compound prepared according to the method described by Greenhill and Tomassini Tetrahydron Lett., 2683-2684 (l974).

Diethyl l-(N-phenylamino)-ethene-l,2-dicarboxylate (l2a): A solution of 3.64 g (0.04 mole) aniline in 20 ml of 90% ethanol was added to a solution of 4.92 ml (0.04 mole) dimethylacetylene dicarboxylate in 20 ml of 90% ethanol at 20° C. This intermediate (l2a) was isolated in 85% yield after removing the solvent by rotary evaporation and used at the starting for the reaction below.

4-(N-Phenylamino)-2(5H)-furanone: A solution of 2 g (0.085 mole) l2a in l0 ml dry tetrahydrofuran (THF) was added dropwise to a suspension of 0.4 g (0.0l05 mole) lithium aluminum hydride (LAH) in 20 ml THF, maintained under gentle reflux. The isolation of the product involved additionof 0.4 ml of water, 0.8 ml l0%

sodium hydroxide and l ml water in the given order to destroy the excess LAH. The suspension was filtered and the title product was isolated after removal of the solvent by rotary evaporation. The product was recrystallized from methanol. Yield, 0.4 g (29%), m.p., 220-221°C.
Anal.---calc. for $C_{10}H_9NO_2$: C, 68.57; H, 5.14; N, 8.00.
Found: C, 68.90; H, 5.33; N, 7.89.
PMR: ($CDCl_3$) 3.33 (s, 2H, $CH_2O$), 5.340 (s, lH, CH=C), 7.30 (m, 5H, Ar-H), 9.55 (broad s, lH, NH) ppm.

EXAMPLE 13

3-(N-Phenylamino-2-cyclopentene-l-one
This compound was prepared from aniline and cyclopentan-l,3-dione using the method described in Example l. Dry benzene was used as a solvent and the reaction was carried out under reflux condition. The product was recrystallized from chloroform. Yield, 57%; m.p., 225°C.
Anal.---calc. for $C_{11}H_{11}NO$: C, 76.28; H, 6.40; N, 8.09.
Found: C, 74.48; H, 6.50; n, 8.00.
PMR: (DMSO-d6): 2.00 (s, 2H, $CH_2CO$), 2.83 (s, 2H, $CH_2$=C), 5.93 (s, lH, CH=C), 7.20 (broad s, 5H, Ar-H), 8.93 (broad s, lH, NH) ppm.

EXAMPLE 14

5,5-Dimethyl-4-(N-phenylamino)-2(5H)-furanone
5,5-Dimethyl-2,4 (3H,5H)-furandione was synthesized from 3-hydroxy-3-methyl-2-butanone by the method of Jerris et al. Tetrahedron Lett., 4517-4520 (1979), in 27% yield, m.p., 140-141° C. The structure was confirmed by p.m.r.
The title compound was prepared via a method similar to the one described for the Example l. Chloroform was used as a solvent and the product was recrystallized from acetone. Yield, 95%; m.p., 253° C.
Anal.---calc. for $C_{12}H_{13}NO_2$: C, 70.93; H, 6.40; N, 6.89.
Found: C, 69.68; H, 6.10; N, 6.89.
PMR: (DMSO-d6) 1.55 (s, 6H, $(CH_3)_2C$), 5.10 (s, lH, CH=C), 7.20 (broad s, 5H, Ar-H), 9.55 (broad s, lH, NH) ppm.

EXAMPLE 15

N-(l-Methyl-3-oxo-l-butenyl)-D,L-valine
A suspension of D,L-valine and pentane-2,4-dione was reacted according to the method by Stork et al., J. Am. Chem. Soc. 76, 2029-2030, as described in Example 4. The product was isolated from the filtrate after the removal of unreacted D,L-valine. Yield, 88%; m.p., 134-135°C.
Anal.---calc. $C_{10}H_{17}NO_3$: C, 60.30; H, 8.54; N, 7.03.
Found: C, 59.9; H, 8.68; N, 6.98.
PMR: ($CDCl_3$) 1.05 (d, 6H, $(CH_3)_2C$, J=6, 6 Hz), 1.92 (s, 3H, $CH_3C$=C), 2.05 (s, 3H, $CH_3C$=O), 2.25 (m, lH, C-CH-C, J=6.6 Hz), 3.9 (m, lH, CH-CH(NH)-CO-), 5.04 (s, lH, CH=C), 9.28 (broad s, lH, NH), 10.96 (broad s, lH, COOH) ppm.

EXAMPLE 16

Methyl 5-Hydroxy-6-methyl-3-(N-phenylamino)-2-heptene-l-carboxylate
Methyl 5-hydroxy-6-methyl-3-oxo-heptane carboxylate (synthesis has been described in Example 9) in dry ether according to the method described in Example 4. The reaction was incomplete even after 40 hours. Tlc (silica, diethyl ether) showed two products apart from the reactants. The mixture of products was separated from the reactants by chromatography (PCTLC[2], silica, 2mm, methylene chloride). The two products could not be separated. The p.m.r. confirmed the title compound as a major component.

**Claims**

l. A compound of formula I or II:

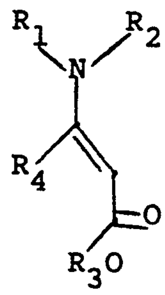 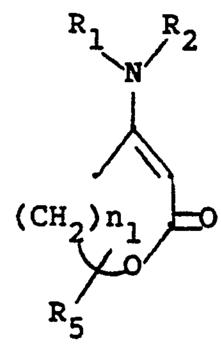 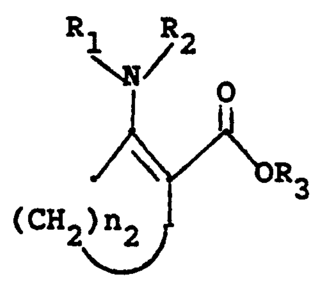

I II III

wherein $n_1$ is I or 2; $n_2$ is 3 or 4; $R_1R_2N$-H is any drug, pharmaceutical or medicament having a primary or secondary amine function; $R_3$ and $R_4$ are the same or different and is alkyl($C_1$-$C_6$), aryl($C_6$-$C_{10}$), aralkyl($C_7$-$C_{12}$), cycloalkyl ($C_3$-$C_8$), cycloalkenyl($C_3$-$C_{10}$), alkenyl($C_2$-$C_{20}$), saturated or unsaturated mono or poly-heterocyclic having from I to 3 rings and having one or more nitrogen, oxygen or sulfur atoms; and $R_5$ is hydrogen or alkyl($C_1$-$C_8$).

2. The compound of Claim I, wherein n is 2; $R_3$ and $R_4$ is selected from the group consisting of alkyl, aryl, saturated and unsaturated mono- or poly-heterocyclic selected from the group consisting of morpholinyl, pyrolyl, 4-methyl-I-piperazinyl, imidazolyl and I,2,5-thiazolyl; and $R_5$ is alkyl.

3. The compound of Claim 2, which is ethyl 2-(N-phenylamino)-I-cycloherene-I-carboxylate, ethyl 3-(N-phenylamino)-2-butenoate, 3-[I-(N-phenylamino)-ethylidene]-4,5-dihydro-2(3H)-furanone, 4-(N-phenylamino)-3-pentene-2-one, ethyl 2-(N-phenylamino)-I-cyclopentene-I-carboxylate, 4-(2-N-phenethylamino)-3-pentene-2-one, 3-(N-phenylamino)-2-cyclohexene-I-one, 5,6-dihydro-6-(I-methylethyl)-4-(N-phenylamino)-2-pyrone, 5,6-dihydro-6-methyl-4-(N-phenylamino)-2-pyrone, 5,5-dimethyl-3-(N-phenylamino)-2-cyclohexane-I-one, 4-(N-phenylamino)-2(5H)-furanone, 3-(N-phenylamino)-2-cyclopentene-I-one, 5,5-dimethyl-4-(N-phenylamino)-2(5H)-furanone, N-(I-methyl-3-oxo-I-butenyl)-D,L-valine or methyl 5-hydroxy-6-methyl-3-(N-phenylamino)-2-heptene-I-carboxylate.

4. A composition useful for administration to a patient requiring medical treatment, comprising an effective amount of a compound of Claim I and a pharmaceutical acceptable carrier.

5. The composition of Claim 4, wherein said compound is ethyl 2-(N-phenylamino)-I-cycloherene-I-carboxylate, ethyl 3-(N-phenylamino)-2-butenoate, 3-[I-(N-phenylamino)-ethylidene]-4,5-dihydro-2(3H)-furanone, 4-(N-phenylamino)-3-pentene-2-one, ethyl 2-(N-phenylamino)-I-cyclopentene-I-carboxylate, 4-(2-N-phenethylamino)-3-pentene-2-one, 3-(N-phenylamino)-2-cyclohexene-I-one, 5,6-dihydro-6-(I-methylethyl)-4-(N-phenylamino)-2-pyrone, 5,6-dihydro-6-methyl-4-(N-phenylamino)-2-pyrone, 5,5-dimethyl-3-(N-phenylamino)-2-cyclohexane-I-one, 4-(N-phenylamino)-2(5H)-furanone, 3-(N-phenylamino)-2-cyclo pentene-I-one, 5,5-dimethyl-4-(N-phenylamino)-2(5H)-furanone, N-(I-methyl-3-oxo-I-butenyl)-D,L-valine or methyl 5-hydroxy-6-methyl-3-(N-phenylamino)-2-heptene-I-carboxylate.